# EUROPÄISCHE PATENTSCHRIFT

(11) **EP 1 698 322 B1**
(45) Veröffentlichungstag und Bekanntmachung des Hinweises auf die Patenterteilung: **16.03.2011**
(21) Anmeldenummer: 06004254.6
(22) Anmeldetag: 02.03.2006
(51) Int. Cl.: A61K 8/36, A61K 8/49, A61K 8/92, A61Q 19/00

(54) **Kosmetische Zusammensetzung enthaltend Allantoin, gamma-Linolensäure, ätherische Öle**
Cosmetic composition containing allantoin, gamma linolenic acid and essential oils
Composition contenant de l'allantoine, l'acide gamma-linolenique et des huiles essentielles

(30) Priorität: 02.03.2005 DE 102005009520; 20.04.2005 DE 202005006334 U
(43) Veröffentlichungstag der Anmeldung: 06.09.2006
(73) Patentinhaber: Sixtuswerke Fritz Becker GmbH & Co., 83727 Schliersee (DE)
(72) Erfinder: Becker, Fritz, 83714 Miesbach-Wachlehen (DE); Becker, Franz, 83727 Schliersee (DE)
(74) Vertreter: Weiss, Wolfgang

(56) Entgegenhaltungen:
- WO-A-00/00186
- DE-U1- 20 209 757
- US-A- 4 707 354
- US-A- 5 011 855
- ANONYMOUS: "Skin Care Treatments" INTERNET ARTICLE, [Online] 3. März 2001 (2001-03-03), XP002382761 Gefunden im Internet: URL:http://web.archive.org/web/20010303111 031/http://abratherapeutics.com/ingredient s/treatments.html> [gefunden am 2005-05-26]
- DATABASE WPI Section Ch, Week 199844 Derwent Publications Ltd., London, GB; Class B04, AN 1998-521648 XP002174586 & ZA 9 610 264 A (PRETORIUS M L E) 26. August 1998 (1998-08-26)
- PATENT ABSTRACTS OF JAPAN Bd. 013, Nr. 472 (C-647), 25. Oktober 1989 (1989-10-25) & JP 01 186809 A (SUNSTAR INC), 26. Juli 1989 (1989-07-26)

## Beschreibung

Die vorliegenden Erfindung betrifft eine Zusammensetzung, insbesondere in Form eines kosmetischen Präparats, die als Wirksubstanzen Allantoin, y-Linolensäure und ätherische Öle enthält.

Der Gegenstand der Erfindung ist durch die Ansprüche 1-13 charakterisiert.

Die erfindungsgemäße Zusammensetzung liegt vorzugsweise als Creme, Shampoo, Lotion, Gel, Flüssigkeit oder Aerosol vor und enthält neben den Wirksubstanzen kosmetisch übliche Bestandteile, wie z.B. Wasser, Alkohole, wie Ethanol, Glycerin oder/und Ethylenglykol, Fette, z.B. Hirschtalg, Öle, z.B. pflanzliche Öle, Emulgatoren, Gelbildner, Konservierungsmittel, Aromen oder/und Füllstoffe.

Die Zusammensetzung eignet sich zur Hautpflege, beispielsweise zur Behandlung von Gesicht, Körper, Händen, Kopfhaut und/oder Füßen. Die Zusammensetzung kann ein- oder mehrmals täglich für eine beliebige Zeitspanne angewendet werden, ohne dass merkbare Nebenwirkungen auftreten. Insbesondere ist die Zusammensetzung für eine Langzeitbehandlung, z.B. eine tägliche Anwendung für einen Zeitraum von 3 Monaten oder mehr geeignet. Eine besonders bevorzugte Anwendung der Zusammensetzung ist die Behandlung von trockener Haut, z.B. im Gesicht. Eine weitere besonders bevorzugte Anwendung ist die Behandlung vo6n Hornhautbildung oder Schrunden an Händen oder Füßen.

Wirksubstanz (a) der Zusammensetzung ist Allantoin (5-Ureidohydantoin), das üblicherweise aus Pflanzen oder synthetisch durch Oxidation von Harnsäure gewonnen wird. Die Zusammensetzung enthält Allantoin in einem Anteil von 0,1-5 Gew.-%, vorzugsweise von 0,2-3 Gew.-% und besonders bevorzugt von 0,4-1,5 Gew.-% auf Basis des Gesamtgewichts der Zusammensetzung.

Wirksubstanz (b) ist y-Linolensäure (Z-6,9,12-Octadecatriensäure) bzw. ein physiologisch verträglicher Ester, z.B. ein Glycerinester, davon. γ-Linolensäure kommt im Samenöl von Nachtkerze (Oenothera biennis), Schwarzer Johannisbeere (Ribes nigrum), Borretsch (Borrago officinalis) und anderen Rauhblattbewächsen sowie in Algenpilzen (Phytomycetes) vor. Eine bevorzugte Quelle für die γ-Linolensäure ist das Nachtkerzenöl, wo sie in einem Anteil von ca. 10 Gew.-% vorhanden ist. Die Zusammensetzung enthält γ-Linolensäure in einem Anteil von 0,05-1 Gew.-%, vorzugsweise von 0,1-0,5 Gew.-% und besonders bevorzugt von 0,15-0,3 Gew.-% auf Basis des Gesamtgewichts der Zusammensetzung. Dies entspricht einem Anteil von Nachtkerzenöl von üblicherweise 0,5-10 Gew.-%, vorzugsweise von 1-5 Gew.-% und besonders bevorzugt von 1,5-3 Gew.-% auf Basis des Gesamtgewichts der Zusammensetzung. Falls die Zusammensetzung ein anderes Material mit γ-Linolensäure enthält, kann sich - entsprechend dem dort vorhandenen Anteil an γ-Linolensäure - ein geänderter Anteil für dieses Material in der Zusammensetzung ergeben.

Wirksubstanz (c) der Zusammensetzung sind ätherische Öle, die aus pflanzlichen oder/und synthetischen Quellen stammen können. Mischungen mehrerer synthetischer Öle, insbesondere mehrerer pflanzlicher ätherischer Öle sind bevorzugt, da sie gegenüber ätherischen Ölen aus einer einzigen Quelle eine erhöhte Stabilität gegenüber Oxidation aufweisen. Bevorzugt werden die ätherischen Öle ausgewählt aus Lavendelöl, Zitrusfruchtöle, z.B. Orangenöl, Zitronenöl oder Mandarinenöl, Salbeiöl, Koniferenöl, z.B. Fichtennadelöl oder Kiefernnadelöl, Thymianöl, Eukalyptusöl, Bohnenkrautöl, Rosmarinöl, Pelargonienöl und Kombinationen, enthaltend mindestens 2, 3, 4 oder 5 der zuvor genannten Öle.

Die Zusammensetzung enthält die ätherischen Öle in einem Anteil von 0,5-5 Gew.-%, vorzugsweise von 1-3 Gew.-% und besonders bevorzugt von 1,5-2,5 Gew.-% auf Basis des Gesamtgewichts der Zusammensetzung. Überraschenderweise wurde festgestellt, dass die ätherischen Öle bei gleichzeitigem Vorhandensein von Allantoin und y-Linolensäure eine die Mikrozirkulation anregende und entzündungshemmende Wirkung besitzen und überraschenderweise keine unerwünschten Nebenwirkungen, insbesondere keine hautreizenden Nebenwirkungen, aufweisen.

Neben den genannten Wirksubstanzen kann die erfindungsgemäße Zusammensetzung noch weitere Wirksubstanzen enthalten, insbesondere entzündungshemmende Wirksubstanzen, wie etwa Pantothenol, Flavonoide oder Flavonole, z.B. aus Extrakten von Kräutern wie Arnika und/oder Kamille, Salicyläure und Kombinationen davon. Insbesondere für die Langzeitbehandlung ist die Zusammensetzung im wesentlichen frei von Harnstoff.

Weiterhin soll die Erfindung durch die nachfolgenden Beispiele näher erläutert werden.

### Beispiel 1: Fußbalsam Schrunden Plus

| **Inhaltsstoffe** | **Anteile** |
|---|---|
| AQUA | A2 |
| ARACHIS HYPOGAEA | C |
| ADEPS CERVI | E |
| CETEARYLALKOHOL | E |
| COCO-GLUCOSID | E |
| LANOLIN | E |
| NATRIUMACRYLAT/ACROLEIN COPOLYMER | E |
| ARNICA MONTANA | F |
| CHAMOMILLA RECUTITA | F |
| ALLANTOIN | F |
| OENOTHERA BIENNIS | F |
| ISOHEXADECAN | F |
| ALCOHOL DENAT. | F |
| DIMETHICON | F |
| SALICYLSÄURE | F |
| LIMONEN | F |
| PANTHENOL | F |
| LAVANDULA ANGUSTIFOLIA | F |
| POLYSORBAT 80 | F |
| ABIES SIBIRICA | F |
| CITRUS LIMONUM | F |
| LINALOOL | F |
| CITRUS BERGAMIA | F |
| CITRUS DULCIS | F |
| PINUS PUMILIO | F |
| GRAMINIS ALPINA | G |
| PELARGONIUM PELTATUM | G |
| ROSMARINUS OFFICINALIS | G |
| EUCALYPTUS GLOBULUS | G |
| CITRONELLOL | G |
| GERANIOL | G |
| BENZYL ALCOHOL | G |
| SATUREIA HORTENSIS | G |
| THYMUS VULGARIS | G |
| CITRAL | G |
| METHYLCHLORISOTHIAZOLINON | G |
| METHYLISOTHIAZOLINON | G |

Anteile: A1 = 75-100 %, A2 = 50-75 %, B = 25-50 %, C = 10-25 %, D = 5-10 %, E = 1-5 %, F = 0,1-1 %, G = 0-0,1 %, H = Spuren

### Beispiel 2: Fußbalsam Forte Plus

| **Inhaltsstoffe** | **Anteile** |
|---|---|
| AQUA | A2 |
| ARACHIS HYPOGAEA | C |
| CETEARYLALKOHOL | E |
| CETYL PALMITAT | E |
| OENOTHERA BIENNIS | E |
| COCO-GLUCOSID | E |
| ARNICA MONTANA | E |
| ALCOHOL DENAT. | F |
| SALICYLSÄURE | F |
| NATRIUMACRYLAT/ACROLEIN COPOLYMER | F |
| BENZOESÄURE | F |
| ALLANTOIN | F |
| ISOHEXADECAN | F |
| LIMONEN | F |
| POLYSORBAT 80 | F |
| LAVANDULA ANGUSTIFOLIA | F |
| MENTHOL | F |
| GRAMINIS ALPINA | F |
| ABIES SIBIRICA | F |
| CITRUS LIMONUM | F |
| LINALOOL | F |
| CITRUS BERGAMIA | F |
| CITRUS DULCIS | F |
| PINUS PUMILIO | G |
| PELARGONIUMPELTATUM | G |
| ROSMARINUS OFFICINALIS | G |
| EUCALYPTUS GLOBULUS | G |
| CITRONELLOL | G |
| GERANIOL | G |
| SATUREIA HORTENSIS | G |
| THYMUS VULGARIS | G |
| CITRAL | G |

Anteile: A1 = 75-100 %, A2 = 50-75 %, B = 25-50 %, C = 10-25 %, D = 5-10 %, E = 1-5 %, F = 0,1-1 %, G = 0-0,1 %, H = Spuren

## Patentansprüche

1. Verwendung einer Zusammensetzung, enthaltend
(a) Allantoin,
(b) γ-Linolensäureoder physiologisch verträgliche Ester hiervon, und
(c) ätherische Öle zur Behandlung von trockener Haut oder Hornhaut , wobei die Zusammensetzung Allantoin in einem Anteil von 0,1-5 Gew.-%, γ-Linolensäure in einem Anteil 0,05-1 Gew.-% und ätherische Öle in einem Anteil von 0,5-5 Gew.-% auf Basis des Gesamtgewichts der Zusammensetzung enthält.

2. Zusammensetzung, enthaltend
(a) Allantoin,
(b) γ-Linolensäure oder physiologisch verträgliche Ester hiervon, und
(c) ätherische Öle
zur Veswendung beider Behandlung von, Schrunden, wobei die Zusammensetzung Allantoin in einem Anteil von 0,1-5 Gew.-%, γ-Linolensäure in einem Anteil 0,05-1 Gew.-% und ätherische Öle in einem Anteil von 0,5-5 Gew.-% auf Basis des Gesamtgewichts der Zusammensetzung enthält.

3. Verwendung nach Anspruch 1, oder 2 **dadurch gekennzeichnet, dass** das Mittel als Creme, Gel, Shampoo, Lotion oder Flüssigkeit vorliegt.

4. Verwendung nach einem der Ausprüche 1 bis 3 zur Behandlung von Gesicht, Körper, Händen, Kopfhaut und/oder Füßen.

5. Verwendung nach einem der Ansprüche 1 bis 4,
**dadurch gekennzeichnet,**
**dass** die Zusammensetzung Allantoin in einem Anteil von 0,2-3 Gew.-%, bevorzugt von 0,4-1,5 Gew.-% auf Basis des Gesamtgewichts der Zusammensetzung enthält.

6. Verwendung nach einem der Ansprüche 1 bis 5,
**dadurch gekennzeichnet,**
**dass** die Zusammensetzung γ-Linolensäure in einem Anteil von 0,1-0,5 Gew.-%, bevorzugt von 0,15-0,3 Gew.-% auf Basis des Gesamtgewichts der Zusammensetzung enthält.

7. Verwendung nach einem der Ansprüche 1 bis 6,
**dadurch gekennzeichnet,**
**dass** die Zusammensetzung γ-Linolensäure in Form von Nachtkerzenöl enthält.

8. Verwendung nach Ansprüche 7,
**dadurch gekennzeichnet,**
**dass** die Zusammensetzung Nachtkerzenöl in einem Anteil von 0,5-10 Gew.-%, vorzugsweise von 1-5 Gew.-% und besonders bevorzugt von 1,5-3 Gew.-% auf Basis des Gesamtgewichts der Zusammensetzung enthält.

9. Verwendung nach einem der Ansprüche 1 bis 8,
**dadurch gekennzeichnet,**
**dass** die Zusammensetzung ätherische Öle in einem Anteil von 1-3 Gew.-%, bevorzugt von 1,5-2,5 Gew.-% auf Basis des Gesamtgewichts der Zusammensetzung enthält.

10. Verwendung nach einem der Ansprüche 1 bis 9,
**dadurch gekennzeichnet,**
**dass** die Zusammensetzung eine Mischung von mehreren ätherischen Ölen enthält.

11. Verwendung nach einem der Ansprüche 1 bis 10,
**dadurch gekennzeichnet,**
**dass** die ätherischen Öle ausgewählt sind aus Lavendelöl, Zitrusfruchtölen, Salbeiöl, Koniferenölen, Thymianöl, Eukalyptusöl, Bohnenkrautöl, Rosmarinöl, Pelargonienöl und Kombinationen, enthaltend mindestens 2 der zuvor genannten Öle.

12. Verwendung nach einem der Ansprüche 1 bis 11,
**dadurch gekennzeichnet,**
**dass** die Zusammensetzung weiterhin mindestens eine entzündungshemmende Wirksubstanz, insbesondere ausgewählt aus Pantothenol, Flavonoiden, Flavonolen, Salicyläure und Kombinationen davon enthält.

13. Zusammensetzung, enthaltend
(a) Allantoin,
(b) γ-Linolensäure oder physiologisch verträgliche Ester hiervon, und
(c) ätherische Öle, wobei die Zusammensetzung Allantoin in einem Anteil von 0,1-1,5 Gew.-%, γ-Linolensäure in einem Anteil 0,15-0,3 Gew.-% und ätherische Öle in einem Anteil von 1,5-2,5 Gew.-% auf Basis des Gesamtgewichts der Zusammensetzung enthält.

## Claims

1. Use of a composition containing
(a) allantoin,
(b) (-linolenic acid or physiologically compatible esters thereof, and
(c) essential oils
for treating dry skin or hard skin, wherein the composition contains allantoin in a proportion of 0.1-5 % by weight, (-linolenic acid in a proportion of 0.05-1 % by weight and essential oils in a proportion of 0.5-5 % by weight based on the total weight of the composition.

2. A composition containing
(a) allantoin,
(b) (-linolenic acid or physiologically compatible esters thereof, and
(c) essential oils
for use in the treatment of chaps, wherein the composition contains allantoin in a proportion of 0.1-5 % by weight, (-linolenic acid in a proportion of 0.05-1 % by weight and essential oils in a proportion of 0.5-5 % by weight based on the total weight of the composition.

3. Use according to Claim 1 or 2,
**characterised in that** the preparation is present in the form of a cream, gel, shampoo, lotion or liquid.

4. Use according to any one of Claims 1 to 3 for treating the face, body, hands, scalp and/or feet.

5. Use according to any one of Claims 1 to 4,
**characterised in that** the composition contains allantoin in a proportion of 0.2-3 % by weight, preferably of 0.4-1.5 % by weight based on the total weight of the composition.

6. Use according to any one of Claims 1 to 5,
**characterised in that** the composition contains (-linolenic acid in a proportion of 0.1-0.5 % by weight, preferably of 0.15-0.3 % by weight based on the total weight of the composition.

7. Use according to any one of Claims 1 to 6,
**characterised in that** the composition contains (-linolenic acid in the form of evening primrose oil.

8. Use according to Claim 7,
**characterised in that** the composition contains evening primrose oil in a proportion of 0.5-10 % by weight, preferably of 1-5 % by weight and especially preferably of 1.5-3 % by weight based on the total weight of the composition.

9. Use according to any one of Claims 1 to 8,
**characterised in that** the composition contains essential oils in a proportion of 1-3 % by weight, preferably of 1.5-2.5 % by weight based on the total weight of the composition.

10. Use according to any one of Claims 1 to 9,
**characterised in that** the composition contains a mixture of several essential oils.

11. Use according to any one of Claim 1 to 10,
**characterised in that** the essential oils are chosen from lavender oil, citrus fruit oil, sage oil, conifer oils, thyme oil, eucalyptus oil, savoury oil, rosemary oil, pelargonium oil and combinations containing at least 2 of the aforementioned oils.

12. Use according to any one of Claims 1 to 11,
**characterised in that** the composition also contains at least one antiinflammatory active substance, in particular chosen from pantothenol, flavonoids, flavonols, salicylic acid and combinations thereof.

13. A composition containing
a) allantoin,
(b) (-linolenic acid or physiologically compatible esters thereof, and
(c) essential oils
wherein the composition contains allantoin in a proportion of 0.1-1.5 % by weight, (-linolenic acid in a proportion of 0.15-0.3 % by weight and essential oils in a proportion of 1.5-2.5 % by weight based on the total weight of the composition.

## Revendications

1. Utilisation d'une composition, contenant :
(a) de l'allantoïne,
(b) de l'acide γ-linolénique ou un ester physiologiquement acceptable de celui-ci, et
(c) des huiles essentielles,
pour le traitement de la peau sèche ou de callosités, la composition contenant de l'allantoïne en une proportion de 0,1-5 % en poids, de l'acide γ-linolénique en une proportion de 0,05-1 % en poids, et les huiles essentielles en une proportion de 0,5-5 % en poids, sur base du poids total de la composition.

2. Composition contenant :
(a) de l'allantoïne,
(b) de l'acide γ-linolénique ou un ester physiologiquement acceptable de celui-ci, et
(c) des huiles essentielles,
à utiliser dans le traitement des gerçures, la composition contenant l'allantoïne en une proportion de 0,1-5 % en poids, l'acide γ-linolénique en une proportion de 0,05-1 % en poids, et les huiles essentielles en une proportion de 0,5-5 % en poids, sur base du poids total de la composition.

3. Utilisation selon la revendication 1 ou 2, **caractérisée en ce que** l'agent se présente sous forme d'une crème, d'un gel, d'un shampoing, d'une lotion ou d'un liquide.

4. Utilisation selon l'une des revendications 1 à 3, pour le traitement du visage, du corps, des mains, de la tête et/ou des pieds.

5. Utilisation selon l'une des revendications 1 à 4, **caractérisée en ce que** la composition contient l'allantoïne en une proportion de 0,2-3 % en poids, de préférence 0,4-1,5 % en poids, sur base du poids total de la composition.

6. Utilisation selon l'une des revendications 1 à 5, **caractérisée en ce que** la composition contient l'acide γ-linolénique en une proportion de 0,1-0,5 % en poids, de préférence 0,15-0,3 % en poids, sur base du poids total de la composition.

7. Utilisation selon l'une des revendications 1 à 6, **caractérisée en ce que** la composition contient l'acide γ-linolénique sous forme d'huile d'onagre.

8. Utilisation selon la revendication 7, **caractérisée en ce que** la composition contient de l'huile d'onagre en une proportion de 0,5-10 % en poids, de préférence 1-5 % en poids et de manière particulièrement préférée, 1,5-3% en poids, sur base du poids total de la composition.

9. Utilisation selon l'une des revendications 1 à 8, **caractérisée en ce que** la composition contient les huiles essentielles en une proportion de 1-3 % en poids, de préférence 1,5-2,5 % en poids, sur base du poids total de la composition.

10. Utilisation selon l'une des revendications 1 à 9, **caractérisée en ce que** la composition contient un mélange de plusieurs huiles essentielles.

11. Utilisation selon l'une des revendications 1 à 10, **caractérisée en ce que** les huiles essentielles sont choisies parmi l'huile de lavande, l'huile d'agrumes, l'huile de sauge, l'huile de conifères, l'huile de thym, l'huile d'eucalyptus, l'huile de sarriette, l'huile de romarin, l'huile de pélargonium et les combinaisons contenant au moins 2 des huiles précédentes.

12. Utilisation selon l'une des revendications 1 à 11, **caractérisée en ce que** la composition contient en outre, au moins une substance anti-inflammatoire, choisie en particulier parmi le pantothénol, les flavonoïdes, les flavonols, l'acide salicylique et leurs combinaisons.

13. Composition contenant:
(a) de l'allantoïne,
(b) de l'acide γ-linolénique ou un ester physiologiquement compatible de celui-ci, et
(c) des huiles essentielles,
la composition contenant l'allantoïne en une proportion de 0,1-1,5 % en poids, l'acide γ-linolénique en une proportion de 0,15-0,3% en poids, et les huiles essentielles en une proportion de 1,5-2,5 % en poids, sur base du poids total de la composition.
